(19) Europäisches Patentamt
European Patent Office
Office européen des brevets

(11) EP 0 947 192 B1

(12) # FASCICULE DE BREVET EUROPEEN

(45) Date de publication et mention de la délivrance du brevet:
**26.11.2003 Bulletin 2003/48**

(51) Int Cl.⁷: **A61K 7/50**

(21) Numéro de dépôt: **99400289.7**

(22) Date de dépôt: **09.02.1999**

(54) **Shampooings antipelliculaires transparents**

Transparente Antischuppen-Haarwaschzusammensetzungen

Transparent antidandruff shampoos

(84) Etats contractants désignés:
**AT BE CH DE DK ES FI FR GB GR IT LI NL PT SE**

(30) Priorité: **13.02.1998 FR 9801771**

(43) Date de publication de la demande:
**06.10.1999 Bulletin 1999/40**

(73) Titulaire: **CECA S.A.**
**92800 Puteaux (Hauts de Seine) (FR)**

(72) Inventeurs:
- **De Mesanstourne, Régine**
  **78600 Maisons-Lafitte (FR)**
- **Le Gall, Hélène**
  **91170 Viry Chatillon (FR)**
- **Lebon, Hélène**
  **78430 Louveciennes (FR)**

(74) Mandataire: **Haicour, Philippe**
**Elf Atochem S.A.,**
**Departement Propriété Industrielle,**
**Cours Michelet-La Défense 10**
**92091 Paris La Défense Cédex (FR)**

(56) Documents cités:
**EP-A- 0 799 612** **WO-A-93/18737**

- **CHEMICAL ABSTRACTS, vol. 123, no. 152594, 18 septembre 1995 Columbus, Ohio, US; abstract no. 152594t, "Cleansing compositions containing modified organosiloxanes" page 595; XP002090620 & JP 07 150190 A (KAO) 13 juin 1995**
- **DATABASE WPI Week 9016 Derwent Publications Ltd., London, GB; AN 90-118686 XP002090621 "Dandruff-resistant hair-washing agent compsn. - contains sulpho-succinate derivs. surfactant(s), amide-betaine amphoteric surfactants and amine oxide surfactant blend" & JP 02 067212 A (SUNSTAR), 7 mars 1990**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 947 192 B1

Printed by Jouve, 75001 PARIS (FR)

## Description

La présente invention concerne de nouvelles compositions utiles en cosmétologie, notamment des shampooings et gels moussants, destinées aux soins des cheveux et/ou du cuir chevelu, en particulier pour combattre les pellicules ou en prévenir la formation. Elle s'adresse aux shampooings dont la base lavante comporte un tensioactif amphotère et un tensioactif anionique, le cas échéant complétée d'un épaississant et/ou d'un conditionneur capillaire.

Dans les shampooings dont la base lavante comporte un tensioactif anionique et un amphotère, la fonction lavante elle-même est dévolue aux deux composants, mais une fonction moussante est assurée par le tensioactif amphotère. D'une façon très générale, le tensioactif amphotère est une alkylbétaïne, en particulier à une alkylamidopropylbétaïne sur base laurique, coprah ou palmiste. L'élimination mécanique des pellicules, dont l'agent responsable est un champignon, le *Pityrosporum ovalae*, est l'un des résultats attendus du shampooinage, mais on a cherché à développer aussi des shampooings qui combattent ou préviennent la formation des pellicules. On a eu recours pour cela à divers agents antifongiques.

Parmi les antifongiques classiques, on peut citer :

- les conservateurs dont le spectre fongique est orienté vers les fongi et particulièrement les levures, tels que l'acide sorbique et l'acide déhydroacétique,
- les actifs répertoriés comme agents antilevures, tels que la zinc pyrithione, la piroctone-olamine et le climbazole,
- quelques tensioactifs dérivés de l'acide undécylénique, en particulier le 4-sulfo-{2-[(1-oxo-10-undecenyl)amino] ethyl}butanedioic ester, disodium (RN=26650-05-5) (ou disodium undecylenamido MEA sulfosuccinate, selon dénomination CTFA empruntée à l'International Cosmetic Ingredient Dictionary, 6ème édition par The Cosmetic, Toiletry, and Flagrance Association, 1995), et le N-(2-Hydroxyethyl)undecenamide (RN=20545-92-0 et 40839-40-5) (CTFA : undecylenamide MEA)

Certains principes actifs antifongiques parmi les plus couramment utilisés présentent toutefois un potentiel cytotoxique in vitro. Il est nécessaire d'en abaisser les concentrations utiles en dessous d'un seuil acceptable cytotoxicité, voire de les associer avec des molécules protégeant l'intégrité des cellules cutanées plus ou moins lésées et enflammées. Ces produits ont d'autres inconvénients : par exemple la piroctone-olamine pose des problèmes de coloration dans les shampooings contenant ce principe actif, la zinc pyrithione est une poudre insoluble dans l'eau qui conduit à des formulations opaques et instables à la lumière, le climbazole est insoluble dans l'eau et nécessite obligatoirement l'utilisation de solvant (éthanol, alcool benzylique).

En ce qui concerne les dérivés de l'acide undécylénique, certains ont déjà été cités pour leur activité antibactérienne et antifongique, notamment les sels de zinc de l'undecylenamido MEA sulfosuccinate (WO 9718823 du 29.05.97 à PIERRE FABRE ; EP 23676 du 11.02.81 à REWO) et du triacide carboxylique issus du produit de réaction Diels-Alder entre l'anhydride maléique et l'acide undécylénique (EP 71025 du 9.02.83 à GRILLO WERKE AG), ainsi que quelques esters (JP 08053326 du 27.02.96 à KANEBO ; EP 28459 du 13.05.81 à IMPERIAL CHEM IND LTD). A titre d'exemples, parmi les dérivés de l'acide undécylénique les plus courants, on trouve le N-(2-Hydroxyethyl)undecenamide (CTFA : undecylenamide MEA), et le 4-sulfo-{2-[(1-oxo-10-undecenyl)-amino]ethyl}butanedioic ester, disodium (CTFA : disodium undecylenamido MEA sulfosuccinate), vendus respectivement sous les dénominations commerciales de "REWOCID®U185 ou WITCAMIDE®6570" et de "REWOCID®SB U185" par la Société WITCO. Ces dérivés ne sont toutefois pas des amphotères.

On a découvert de manière inattendue qu'en plus des caractéristiques usuelles des bétaïnes (co-tensioactif, bon pouvoir moussant, détergent doux), le N-(carboxymethyl)-N,N-dimethyl-3-[(1-oxoundecenyl)amino]-1-propanaminium hydroxyde (RN = 98510-75-9, forme zwitterion neutre) ou CTFA : undecylenamidopropylbetaine

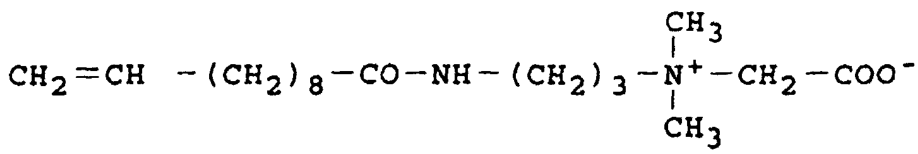

présentait une activité antifongique vis à vis du *Pityrosporum ovalae* et développait donc une efficacité antipelliculaire jamais citée à ce jour. En conséquence, elle offre le très gros avantage de pouvoir constituer un composant amphotère d'un shampooing lui-même doué des propriétés antipelliculaires souhaitées. Cette découverte est exploitée dans les shampooings de l'invention qui sont des compositions essentiellement aqueuses d'une base lavante constituée de tensioactifs amphotères et de tensioactifs anioniques et dont les tensioactifs amphotères sont eux-mêmes - et c'est

la caractéristique de l'invention - constitués en partie d'undécylènamidopropylbétaïne.

Au sens de l'invention, on entend par tensioactif anionique un ou plusieurs composés pris dans le groupe formé des alkyl($C_{10}$ à $C_{14}$)sulfates et les alkyl($C_{10}$ à $C_{14}$) éthersulfates. Les lauryléthersulfates sont des tensioactifs anioniques utilisés de façon courante par les formulateurs de shampooings, et de façon préférée pour la réalisation des compositions selon l'invention.

Au sens de l'invention, on entend par tensioactif amphotère les composés choisis, seuls ou en mélange, parmi les alkylamphocarboxyglycinates, les alkylamphocarboxypropionates, les alkylamphodiacétates, les alkylamphodipropionates, les alkylamphoglycinates, les alkylamphopropionates, les alkyliminopropionates, les alkyliminodipropionates, les alkylamphopropylsulfonates, les alkylbétaïnes, les alkylamidopropylbétaïnes, les alkylsultaïnes, et les alkylamidopropylhydroxysultaïnes. Dans cette liste, alkyl s'entend au sens de chaînes hydrocarbonées $C_{10}$ à $C_{14}$ et plus spécialement de restes d'acides gras laurique, de coprah ou de palmiste ou de sources équivalentes. Les coprahamidopropylbétaïnes sont des amphotères préférés pour la réalisation des formulations selon l'invention.

L'undécylènamidopropylbétaïne qui est l'amphotère caractéristique de l'invention est une alkylamidopropylbétaïne sur base undécylénique. Elle peut être préparée selon des procédés connus de l'homme de l'art, en particulier en suivant la procédure décrite dans le brevet US 3,225,074 (AMERICAN CYANAMID) qui consiste à faire réagir l'acide undécylénique avec la N,N-diméthyl-1,3-propanediamine (DMAPA) entre 140 et 200°C, puis l'amidoamine tertiaire

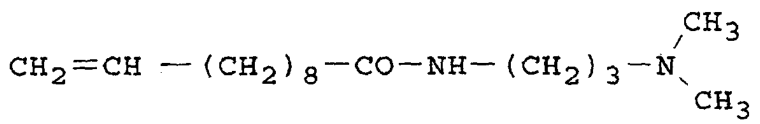

ainsi obtenue avec de l'acide monochloracétique en présence d'un sel alcalin ou le sel correspondant de cet acide, la réaction se faisant en milieu aqueux. Le chlorure alcalin, fréquemment le chlorure de sodium, coproduit pendant la réaction est généralement laissé dans cette solution aqueuse de bétaïne.

Les compositions de l'invention sont des solutions aqueuses dans lesquelles la teneur de l'ensemble des constituants de la base lavante (amphotères et anioniques) se situe entre certaines limites de façon à assurer leur équilibre physique, en particulier leur homogénéité et leur transparence (en l'absence de l'introduction intentionnelle d'agents nacrants), limites naturellement susceptibles de quelques variations selon les composants individuels utilisés, et qu'on lira avec les symboles suivants : An mis pour composants anioniques, Amph pour composés amphotères, ΣAmph représentant l'ensemble des amphotères, AmphU représentant la bétaïne undécylénique, Amph* représentant les composants amphotères à l'exclusion de la bétaïne undécylénique.

On a ainsi d'une part quant à l'équilibre anioniques/amphotères :

$$12{,}5\ \% \leq An + \Sigma Amph \leq 17{,}6$$

$$5\ \% \leq An \leq 15$$

$$3{,}5\ \% \leq \Sigma Amph \leq 9{,}2,$$

d'autre part quant à l'équilibre amphotères / amphotère undécylénique :

$$3{,}5\ \% \leq \Sigma Amph \leq 9{,}2$$

$$2\ \% \leq Amph^* \leq 7$$

$$1{,}5\ \% \leq AmphU \leq 3{,}5$$

Une composition courante de shampooings selon l'invention aura par exemple la composition générale

| | |
|---|---|
| Lauryléthersulfate de sodium | 5 à 15 % |
| Cocoamidopropylbétaïne | 2 à 7 % |
| Undécylènamidopropylbétaïne | 1,5 à 5,0 % |
| Ingrédients ordinaires de formulations de shampooings et eau déminéralisée | QSP 100 |

Ingrédients ordinaires de formulations de shampooings et eau déminéralisée QSP 100

Il entre dans ces compositions les ingrédients courants de la formulation de ce genre de produits, à savoir :

- Des agents épaississants destinés à donner aux shampooings une consistance suffisante et des qualités cosmétiques améliorées. Parmi les agents épaississants connus, les carbomers (homopolymères d'acides acrylique réticulés avec un éther allylique de pentaérythritol, de sucrose ou de propylène), les esters de PEG, la lauryl pyrrolidone, les copolymères acryliques et les polymères cationiques sur base naturelle ou synthétique, y compris certains polyquaternium (voir plus bas, agents conditionneurs), donnent dans l'application considérée, des résultats très satisfaisants, compte tenu notamment du fait que des viscosités supérieures à 1000 mPa.s, de préférence comprise entre 1000 mPa.s et 30000 mPa.s, ont pu être obtenues avec ces produits particuliers ("CARBOPOL ULTREZ® 10" de BF GOODRICH, "ATLAS®G-1821" de ICI, "SURFADONE®LP 300" de ISP, "ACRYSOL®22" de ROHM & HASS, "JAGUAR®C 162" et "RHODICARE®T" de RHÔNE-POULENC, "UCARE®POLYMER Jr. 400" de AMERCHOL, "MACKERNIUM®7 de JAN DEKKER/MAC INTYRE et "GAFQUAT ®755 N" de ISP). Ces agents épaississants spécifiques permettent d'obtenir des viscosités acceptables lorsqu'ils sont utilisés à des concentrations comprises entre 0 et 4 % en poids par rapport à l'ensemble de la composition, de préférence entre 0,2 et de 2 % en poids.
- Des agents conditionneurs, produits destinés à améliorer les propriétés cosmétiques de solution détergente, généralement les polymères cationiques ou amphotères, des huiles, et en particulier des silicones ou dérivés de silicone, dont il convient de vérifier la compatibilité avec l'ensemble des autres constituants de la formulation, et en particulier pour éviter la formation gels troubles, cosmétiquement inacceptables ; à cet égard, il a été trouvé que l'utilisation de polymères cationiques du type polyquaternium 7 (notamment le "MACKERNIUM 7" de JAN DEKKER/MAC INTYRE), polyquaternium 10 (notamment le "UCARE POLYMER JR 400" de AMERCHOL) et polyquaternium 11 (notamment le "GAFQUAT 755 N" de ISP) à des concentrations de l'ordre de 1 à 4 %, permettaient de conserver des gels parfaitement limpides.
- Des promoteurs / stabilisants de mousse, composés pris dans le groupe constitué par l'alcanolamide de diéthanolamine ou les oxydes d'amines tertiaires, qu'on peut utiliser à des teneurs comprises entre 1 et 5 % en poids par rapport au poids total de la composition, mais qu'on a de plus en plus tendance à écarter de la formulation par crainte de toxicité induite par certaines de leurs impuretés possibles.

Elles peuvent en outre contenir certains adjuvants usuels du domaine des shampooings, agents conservateurs, séquestrants, adoucissants, modificateurs de mousse, colorants, agents nacrants, agents hydratants, agents antiséborrhéiques, vitamines, filtres solaires, parfums. A propos des parfums de la formulation, il convient de remarquer que la faible odeur caractéristique de l'undécénylamidopropylbétaïne autorise tout parfum du plus discret au plus spécifique.

L'utilisation seul ou en mélange des agents conservateurs usuels de type phenoxyethanol, methylparaben, ethylparaben, propyl paraben et butylparaben (nomenclatures CTFA, INCI, 6ème édition) à des concentrations comprises entre 0,25 % et 1 % en poids par rapport à l'ensemble de la composition, s'est révélée particulièrement utile pour obtenir une protection efficace et prolongée dans le temps des gels moussants.

Les compositions selon l'invention peuvent également contenir des solvants additionnels, par exemple des glycols ou la glycérine qui, à des concentrations comprises entre 2 % et 8 % en poids, et plus particulièrement encore entre 4 % et 6 % en poids, par rapport à l'ensemble de la composition, améliorent la stabilité physique des formulations aux basses températures (problèmes de trouble ou de croissance de cristaux notamment), et favorisent une pénétration rapide du principe actif dans la couche cornée et d'améliorer l'état de la couche cornée irritée (hydratation des couches superficielles de l'épiderme du cuir chevelu).

Le pH de ces compositions doit se situer entre 4,5 et 7,0, plutôt vers 6, pour que soit préservée au meilleur niveau l'activité antifongique. L'ajustement du pH à la valeur désirée se fait classiquement par addition d'un acide ou d'une base organique ou minérale, par exemple l'acide citrique, l'acide succinique, l'acide phosphorique, la soude, le carbonate de sodium.

Les compositions lavantes et antifongiques selon l'invention sont stables (absence de décantation ou de démixtion de phase) et homogènes au cours du temps dans un intervalle de température compris entre 4 et 45°C tant en lumière naturelle que dans nos conditions de vieillissement au Sun Test. Elles offrent une bonne tolérance cutanée.

Elles présentent en outre un bon pouvoir moussant et lavant, ce qui permet l'élimination des pellicules et une bonne rinçabilité. Leur viscosité doit être suffisante pour qu'elles puissent être dosées facilement dans la main. En pratique, leur viscosité doit être supérieure à 1 000 mPa.s.

Les formulations selon l'invention se prêtent à la réalisation de produits transparents très peu colorés à texture gel ou liquide plus ou moins visqueux.

Pour un traitement curatif des pellicules par voie topique, on préfère les formes gels, qui s'utilisent comme des shampooings, c'est à dire qu'elles sont appliquées sur la peau, les cheveux et/ou le cuir chevelu pendant quelques minutes, puis rincées à l'eau.

Des exemples concrets de formulations vont illustrer l'invention.

EXEMPLES

Les compositions décrites ci-après sont exprimées avec des pourcentages en poids des matières actives. On a précisé les pourcentages en poids des produits bruts ou commerciaux utilisés correspondants.

L'undécylènamidopropylbétaïne utilisée dans ces exemples a été synthétisée selon le mode opératoire décrit plus haut. Sa composition s'établit comme suit :

| | |
|---|---|
| Undécylènamidopropylbétaïne | 30,0 ± 2 % |
| NaCl | 6 % max. |
| Acide undécylénique | 0,5 % max. |
| Amidoamine | 0,5 % max. |
| Monochloracétate de Na | < 20 ppm |

Son pH à 5 % dans l'eau est de 5,8 à 7,5, et sa couleur Hazen inférieure à 300.

Exemple 1 : efficacité comparée de l'undécylènamidopropylbétaïne et de l'Octopyrox.

Les tests d'efficacité antipelliculaire et séborégulatrice se sont déroulés sur une période de 15 jours, sur un Test Panel de 20 personnes dont les caractéristiques démographiques sont les suivantes :

| Age | Nombre | Moyenne | Ecart-type | Age minimum | Age maximum |
|---|---|---|---|---|---|
| Total | 20 | 30 | 9 | 23 | 28 |

a) Action antipelliculaire

On a comparé l'undécylènamidopropylbétaïne (Amphoram®U de CECA S.A.) à la piroctone olamine (CFTA) ou 1-hydroxy-4-methyl-6-(2,4,4-trimethylpentyl)-2(1H)-pyridone ; ethanol (1:1) salt (RN=68890-66-4), prise dans sa forme commerciale d'Octopyrox® (Hoechst), aux doses minimales inhibitrices (C.M.I.) de *Pityrosporum ovalae*, à savoir respectivement préalablement 2% et 0,2%. Les résultats de cette étude comparative, exprimés en pourcentage de variations à J15 par rapport à J1 sont présentés dans le tableau suivant :

| | Surface occupée par les pellicules | | Indice d'élimination des pellicules | |
|---|---|---|---|---|
| Produits | Amphoram U | Octopyrox | Amphoram U | Octopyrox |
| Résultats | -29 | -7 | -36 | -5 |

b) Résultats de sébumétrie

Les résultats de l'indice de sébumétrie mesuré avec le sébumètre commercialisé par la Société COURAGE-KHAZKA, exprimés en pourcentage de variation à J15 par rapport à J1 sont présentés dans le tableau suivant :

| | Indice de sébumétrie | |
|---|---|---|
| Produits | AMPHORAM U | OCTOPYROX |
| Résultats | -7 | -4 |

De cet ensemble de résultats, on conclut :

- que l'état pelliculaire des 20 sujets s'est amélioré (baisse d'environ 30 à 40% de la surface occupée et relèvement de l'indice d'élimination des pellicules) avec l'Amphoram U, alors qu'il est resté stable avec l'Octopyrox;
- que le taux de sébumétrie n'a pas varié de façon significative, quel que soit le produit utilisé, ce qui démontre l'absence d'irritation.

Aucun effet secondaire n'a été enregistré quel que soit le produit utilisé.

Exemple 2 : Shampooing antipelliculaire visqueux et transparent

| | |
|---|---|
| Lauryléthersulfate de sodium (1) | 8,4 |
| Cocoamidopropylbétaïne (2) | 3,0 |
| Undécylènamidopropylbétaïne (3) | 2,0 |
| Laurylamide de diéthanolamine (4) | 2,0 |
| Conservateur, parfum | QS |
| Acide citrique | QS |
| Eau déminéralisée | QSP 100 |

*Produits commerciaux utilisés*
*(1) Empicol® ESB 3M (ALBRIGHT & WILSON) pour 28 %*
*(2) Amphoram® C 30 (CECA) pour 10 %*
*(3) Amphoram® U (CECA) 6,6 %*
*(4) Mackamide® LMD (MAC INTYRE) pour 2 %*

Caractéristiques

- Shampooing visqueux (viscosité au viscosimètre Brookfield R5 V10 de 12 800 mPa.s), s'écoulant bien d'un flacon, s'étalant bien sur les cheveux, totalement transparent et incolore (couleur = 40 Hazen), pH = 6,6.
- Formule stable, testée à + 4°C et + 45°C (exposition pendant 1 mois à la lumière naturelle derrière une vitre, 310 nm) et Suntest (15 jours en vieillissement accéléré en appareillage de type suntest, puissance 500 watt/$m^2$, 300 à 800 nm);
- Forme une belle mousse blanche abondante et stable, à rinçage facile.

Exemple 3 : Shampooing antipelliculaire transparent et très visqueux pour pellicules rebelles

| | |
|---|---|
| Lauryléthersulfate de sodium (1) | 8,4 |
| Cocoamidopropylbétaïne (2) | 4,5 |
| Undécylènamidopropylbétaïne (3) | 3,0 |
| Laurylamide de diéthanomamine (4) | 2,0 |
| Conservateur, Parfum | QS |
| Acide citrique | QS |
| Eau déminéralisée | QSP 100 |

*Produits commerciaux utilisés*
*(1) Empicol ® ESB 3M (ALBRIGHT & WILSON) pour 28 %*
*(2) Amphoram ® C 30 (CECA) pour 15 %*
*(3) Amphoram ® U (CECA) 9,9 %*
*(4) Mackamide ® LMD (MAC INTYRE) pour 2 %*

Caractéristiques

- Shampooing très visqueux (viscosité au viscosimètre Brookfield R5 V10 = 31 000 mPa.s)
- Totalement transparent et incolore (couleur : 60 Hazen).
- pH : 6,1.
- Formule stable, testée à + 4°C et 45°C (1 mois) et Suntest (15 h) .
- Forme une belle mousse blanche abondante et stable, rinçage facile.

Exemple 4 : Shampooing antipelliculaire transparent peu visqueux

| | |
|---|---|
| Lauryléthersulfate de sodium (1) | 7,5 |
| Cocoamidopropylbétaïne (2) | 3,0 |
| Undécylènamidopropylbétaïne (3) | 2,0 |
| Conservateur, parfum | QS |
| Chlorure de sodium | 1,0 |
| Eau déminéralisée | QSP 100 |

*Produits commerciaux utilisés*
*(1) Empicol ®ESB 3M (ALBRIGHT & WILSON) pour 25 %*
*(2) Amphoram ®C 30 (CECA) pour 6,6 %*
*(3) Amphoram ®U (CECA) 9,9 %*

Caractéristiques :

- Shampooing fluide totalement transparent et incolore
- S'écoule bien d'un flacon, s'étale bien sur les cheveux
- Belle mousse blanche abondante et stable, rinçage facile
- Formule stable, testée à + 4°C et + 45°C (1 mois en lumière naturelle) et Suntest (15 h 00 au Suntest)
- pH : 6,2
- Viscosité (Brookfield R5 V100): 1 040 mPa.s
- Couleur : 129 Hazen

Exemple 5 : Shampooing antipelliculaire transparent et visqueux

| | |
|---|---|
| Lauryléthersulfate de sodium (1) | 8,4 |
| Cocoamidopropylbétaïne (2) | 4,5 |
| Undécylènamidopropylbétaine (3) | 2,0 |
| Chlorure de sodium | 0,5 |
| Conservateur, Parfum | QS |
| Eau déminéralisée | QSP 100 |

*Produits commerciaux utilisés*
*(1) Empicol® ESB 3M (ALBRIGHT & WILSON) pour 28 %*
*(2) Amphoram® C 30 (CECA) pour 15 %*
*(3) Amphoram® U (CECA) 6,6 %*

Caractéristiques :

- Shampooing visqueux totalement transparent et incolore
- S'écoule bien d'un flacon, s'étale bien sur les cheveux
- Belle mousse blanche abondante et stable, rinçage facile
- Formule stable, testée à + 4°C et + 45°C (1 mois en lumière naturelle) et Suntest (15 h 00 au Suntest)
- pH : 6,2
- Viscosité (Brookfield R5 V10) : 10 600 mPa.s
- Couleur : 88 Hazen

Exemple 6 : Shampooing antipelliculaire transparent peu visqueux

| | |
|---|---|
| Lauryléthersulfate de sodium (1) | 6,3 |
| Cocoamidopropylbétaïne (2) | 6,3 |
| Undécylènamidopropylbétaïne (3) | 2,0 |
| Conservateur, parfum | QS |
| Eau déminéralisée | QSP 100 |

*Produits commerciaux utilisés*
*(1) Empicol ® ESB 3M (ALBRIGHT & WILSON) pour 25 %*
*(2) Amphoram ® C 30 (CECA) pour 15 %*
*(3) Amphoram ® U (CECA) 6,6 %*

Caractéristiques

- Shampooing fluide transparent et incolore
- S'écoule bien du flacon, s'étale bien sur les cheveux
- Belle mousse blanche abondante et stable, rinçage facile
- Formule stable, testée à + 4°C et + 45°C (1 mois en lumière naturelle) et Suntest (15 h 00 au Suntest)
- pH : 6,0
- Viscosité (Brookfield R5 V100) : 2 300 mPa.s
- Couleur : 50 Hazen

Exemple 7 : Shampooing antipelliculaire opaque, gélifié et visqueux

| | |
|---|---|
| Lauryléthersulfate de sodium (1) | 7,5 |
| Cocoamidopropylbétaïne (2) | 4,5 |
| Undécylènamidopropylbétaïne (3) | 2,0 |
| Carbomer (5) | 0,9 |
| Triéthanolamine à 99% | 1,0 |
| Conservateur, parfum | QS |
| Eau déminéralisée | QSP 100 |

*Produits commerciaux utilisés*
*(1) Empicol ® ESB 3M (ALBRIGHT & WILSON) pour 25 %*
*(2) Amphoram ® C 30 (CECA) pour 15 %*
*(3) Amphoram ® U (CECA) 6,6 %*
*(5) Carbopol Ultrez ®1,0 %*

Caractéristiques :

- Shampooing visqueux opaque
- S'écoule bien du flacon, s'étale bien sur les cheveux
- Belle mousse blanche abondante et stable, rinçage facile
- Formule stable, testée à + 4°C et + 45°C (1 mois en lumière naturelle) et Suntest (15 h 00 au Suntest)
- pH : 5,1
- Viscosité (Brookfield R5 V10) : 10 500 mPa.s

Exemple 8 : Shampooing antipelliculaire soyeux et blanc nacré

| | |
|---|---|
| Lauryléthersulfate de sodium (1) | 7,5 |
| Cocoamidopropylbétaïne (2) | 4,5 |
| Undécylènamidopropylbétaïne (3) | 2,0 |
| Carbomer (5) | 0,9 |
| Glycol distéarate (6) | 2,0 |
| Triéthanolamine à 99% | 1,0 |
| Conservateur, Parfum | QS |
| Eau déminéralisée | QSP 100 |

*Produits commerciaux utilisés*
*(1) Empicol ® ESB 3M (ALBRIGHT & WILSON) pour 25 %*
*(2) Amphoram ® C 30 (CECA) pour 15 %*
*(3) Amphoram ® U (CECA) 6,6 %*
*(5) Carbopol Ultrez ® 1,0 %*
*(6) Cutina ® AGS 2,0 %*

Caractéristiques :

- Shampooing blanc nacré, bel aspect riche, laiteux et visqueux
- S'écoule bien du flacon, s'étale bien sur les cheveux
- Belle mousse blanche dense, rinçage facile
- Formule stable, testée à + 4°C et + 45°C (1 mois en lumière naturelle) et Suntest (15 h 00 au Suntest)
- pH : 5,1
- Viscosité (Brookfield R5 V10) : 20 000 mPa.s

Exemple 9 : Shampooing antipelliculaire gélifié, transparent et fluide

| | |
|---|---|
| Lauryléthersulfate de sodium (1) | 7,5 |
| Cocoamidopropylbétaïne (2) | 3,0 |
| Undécylènamidopropylbétaïne (3) | 2,0 |
| Copolymère d'acide acrylique et d'acrylate d'alcool gras $C_{20}$ (7) | 0,6 |
| Hydroxyde de sodium | 0,1 |
| Conservateur, Parfum | QS |

Produits commerciaux *utilisés*
*(1) Empicol ® ESB 3M (ALBRIGHT & WILSON) pour 25 %*
*(2) Amphoram ® C 30 (CECA) pour 10 %*
*(3) Amphoram ® U (CECA) 6, 6 %*
*(7) Acrysol ® 22 pour 2,0 %*

(suite)

| | |
|---|---|
| Eau déminéralisée | QSP 100 |

Caractéristiques :

- Shampooing fluide translucide
- S'écoule bien du flacon, s'étale bien sur les cheveux
- Belle mousse blanche abondante et stable, rinçage facile
- Formule stable, testée à + 4°C et + 45°C (1 mois en lumière naturelle) et Suntest (15 h 00 au Suntest)
- pH : 6 , 5
- Viscosité (Brookfield R5 V50) : 3 700 mPa.s
- Couleur : 204 Hazen

Exemple 10 : Shampooing antipelliculaire transparent et peu visqueux

| | |
|---|---|
| Lauryléthersulfate de sodium (1) | 7,5 |
| Cocoamidopropylbétaïne (2) | 3,0 |
| Undécylènamidopropylbétaïne (3) | 2,0 |
| Polyquaternium 10(8) | 2,0 |
| Conservateur, parfum | QS |
| Eau déminéralisée | QSP 100 |

*Produits commerciaux utilisés*
*(1) Empicol ® ESB 3M (ALBRIGHT & WILSON) pour 25 %*
*(2) Amphoram ® C 30 (CECA) pour 10 %*
*(3) Amphoram ® U (CECA) 6,6 %*
*(8) UCARE ® POLYMER JR 400 pour 2,0 %*

Caractéristiques :

- Shampooing fluide translucide,
- S'écoule bien du flacon, s'étale bien sur les cheveux,
- Belle mousse blanche abondante et stable, rinçage facile
- Formule stable, testée à + 4°C et + 45°C (1 mois en lumière naturelle) et Suntest (15 h 00 au Suntest)
- pH : 6,2
- Viscosité (Brookfield R5 V100) : 1 880 mPa.s
- Couleur : 78 Hazen

Exemple 11 : Shampooing doux antipelliculaire transparent et peu visqueux *(8/2 = HB 1.122 A)*

| | |
|---|---|
| Lauryléthersulfate de sodium (1) | 12,0 |
| Cocoamidopropylbétaïne (2) | 3,6 |
| Undécylènamidopropylbétaïne (3) | 2,0 |

*Produits commerciaux utilisés*
*(1) Empicol ® ESB 3M (ALBRIGHT & WILSON) pour 40 %*
*(2) Amphoram ® C 30 (CECA) pour 12 %*
*(3) Amphoram ® U (CECA) 6,6 %*

(suite)

| | |
|---|---|
| Polyquaternium 7(9) | 0,3-0,4 |
| Conservateur, parfum | QS |
| Eau déminéralisée | QSP 100 |

*(9) MACKERNIUM ® 7 pour 4,0 %*

Caractéristiques :

- Shampooing fluide transparent et incolore
- S'écoule bien du flacon, s'étale bien sur les cheveux
- Belle mousse douce, rinçage facile
- Formule stable, testée à + 4°C et + 45°C (1 mois en lumière naturelle) et Suntest (15 h 00 au Suntest)
- pH : 6.7
- Viscosité (Brookfield R5 V100) : 1 400 mPa.s
- Couleur : 44 Hazen

Exemple 12 : Shampooing blanc nacré antipelliculaire très doux à usage fréquent.

| | |
|---|---|
| Lauryléthersulfate de sodium (1) | 9,0 |
| Cocoamidopropylbétaïne (2) | 2,4 |
| Undécylènamidopropylbétaïne (3) | 2,0 |
| Méthylcocoyl taurate de sodium (10) | 3,0 |
| Glycol distéarate (6) | 2,0 |
| Polyquaternium 11(11) | 0,4 |
| Laurylpyrrolidone(12) | 2,0 |
| Conservateur, parfum | QS |
| Eau déminéralisée | QSP 100 |

Produits commerciaux utilisés
*(1) Empicol ® ESB 3M (ALBRIGHT & WILSON) pour 30 %*
*(2) Amphoram ® C 30 (CECA) pour 8, 0 %*
*(3) Amphoram ® U (CECA) 6,6 %*
*(9) MACKERNIUM ® 7 pour 4,0 %*
*(10) Cutina ® AGS 2, 0 %*
*(11) GAFQUAT ® 755 N pour 2,0 %*
*(12) Surfadone ® LP 300 pour 2,0 %*

Caractéristiques :

- Shampooing visqueux blanc nacré
- S'écoule bien du flacon, s'étale bien sur les cheveux
- Belle mousse blanche , rinçage facile
- Formule stable, testée à + 4°C et + 45°C (1 mois en lumière naturelle) et Suntest (15 h 00 au Suntest)
- pH : 6,6
- Viscosité (Brookfield R5 V10) : 18 300 mPa.s

## Revendications

**1.** Compositions aqueuses de shampooings antifongiques et antipelliculaires comportant une base lavante constituée d'un tensioactif anionique et d'un tensioactif amphotère, dans lesquelles le tensioactif anionique est pris, seul ou

en mélange, dans le groupe constitué des alkyl($C_{10}$ à $C_{14}$)sulfates et les alkyl($C_{10}$ à $C_{14}$)éthersulfates et le tensioactif amphotère est pris, seul ou en mélange, dans le groupe constitué des alkylamphocarboxyglycinates, des alkylamphocarboxypropionates, des alkylamphodiacétates, des alkylamphodipropionates, des alkylamphoglycinates, des alkylamphopropionates, des alkyliminopropionates, des alkyliminodipropionates, des alkylamphopropylsulfonates, des alkylbétaïnes, des alkylamidopropylbétaïnes, des alkylsultaïnes, et des alkylamidopropylhydroxysultaïnes,
**caractérisées en ce qu'**une partie du tensioactif amphotère est une undécylènamidopropylbétaïne.

2. Compositions aqueuses de shampooings selon la revendication 1, dont le tensioactif anionique est un lauryléthersulfate et le tensioactif amphotère est une coprah-amidopropylbétaïne.

3. Compositions aqueuses de shampooings selon les revendications 1 ou 2, dans lesquelles lesquels les composants anioniques et amphotères sont répartis,
d'une part quant à l'équilibre anioniques / amphotères selon les inéquations:

$$12{,}5\ \% \leq An + \Sigma Amph \leq 17{,}6$$

$$5\ \% \leq An \leq 15$$

$$3{,}5\ \% \leq \Sigma Amph \leq 9{,}2,$$

d'autre part quant à l'équilibre amphotères / amphotère undécylénique selon les inéquations:

$$3{,}5\ \% \leq \Sigma Amph \leq 9{,}2$$

$$2\ \% \leq Amph^* \leq 7$$

$$1{,}5\ \% \leq AmphU \leq 3{,}5$$

inéquations dans lesquelles
An est mis pour composants anioniques, Amph pour composés amphotères,
ΣAmph représente l'ensemble des amphotères,
AmphU représente l'undécylènamidopropylbétaïne,
Amph* représente les composants amphotères à l'exclusion de l'undécylènamidopropylbétaïne.

4. Composition aqueuse de shampooing antifongique et antipelliculaire avec base lavante constituée d'un tensioactif anionique et d'un tensioactif amphotère dont une partie est l'undécylènamidopropylbétaïne, répondant à la formule

| | |
|---|---|
| Lauryléthersulfate de sodium | 5 à 15 % |
| Cocoamidopropylbétaïne | 2 à 7 % |
| Undécylènamidopropylbétaïne | 1,5 à 5,0 % |
| Ingrédients ordinaires de formulations de shampooings et eau déminéralisée (pour-cent en poids par rapport à la composition). | QSP 100 |

5. Utilisation des compositions de shampooings selon l'une ou l'autre des revendications précédentes pour le traitement cosmétique de la formation de pellicules dues à l'action du *Pityrosporum ovalae*.

## Patentansprüche

1. Wässerige Haarwaschmittelzusammensetzungen gegen Pilze und gegen Schuppen, die einen Waschrohstoff enthalten, der aus einem anionischen grenzflächenaktiven Stoff und einem amphoteren grenzflächenaktiven Stoff besteht, worin der anionische grenzflächenaktive Stoff allein oder im Gemisch unter $C_{10-14}$-Alkylsulfaten und $C_{10-14}$-Alkylethersulfaten ausgewählt ist und der amphotere grenzflächenaktive Stoff allein oder im Gemisch unter Alkylamphocarboxyglycinaten, Alkylamphocarboxypropionaten, Alkylamphodiacetaten, Alkylamphodipropionaten, Alkylamphoglycinaten, Alkylamphopropionaten, Alkyliminopropionaten, Alkyliminodipropionaten, Alkylamphopropylsulfonaten, Alkylbetainen, Alkylamidopropylbetainen, Alkylsultainen und Alkylamidopropylhydroxysultainen ausgewählt ist, **dadurch gekennzeichnet, dass** der amphotere grenzflächenaktive Stoff zum Teil aus einem Undecylenamidopropylbetain besteht.

**2.** Wässerige Haarwaschmittelzusammensetzungen nach Anspruch 1, worin der anionische grenzflächenaktive Stoff ein Laurylethersulfat und der amphotere grenzflächenaktive Stoff ein Kopraamidopropylbetain ist.

**3.** Wässerige Haarwaschmittelzusammensetzungen nach den Ansprüchen 1 oder 2, worin die anionischen und amphoteren Bestandteile einerseits in Hinblick auf die anionischen Bestandteile/amphoteren Bestandteile den folgenden Ungleichungen:

$$12{,}5\ \% \leq An + \Sigma Amph \leq 17{,}6$$

$$5\ \% \leq An \leq 15$$

$$3{,}5\ \% \leq \Sigma Amph \leq 9{,}2$$

und andererseits in Hinblick auf die amphoteren Bestandteile/amphotere Undecylenverbindung den folgenden Ungleichungen:

$$3{,}5\ \% \leq \Sigma Amph \leq 9,\ 2$$

$$2\ \% \leq Amph^* \leq 7$$

$$1{,}5\ \% \leq AmphU \leq 3{,}5$$

entsprechen, wobei in den Ungleichungen:

- An für die anionischen Bestandteile und Amph für die amphoteren Verbindungen steht,
- ΣAmph die Gesamtheit der amphoteren Bestandteile bedeutet,
- AmphU Undecylenamidopropylbetain bedeutet und
- Amph* die amphoteren Bestandteile mit Ausnahme von Undecylenamidopropylbetain bedeutet.

**4.** Wässerige Haarwaschmittelzusammensetzung gegen Pilze und gegen Schuppen mit einem Waschrohstoff, der aus einem anionischen grenzflächenaktiven Stoff und einem amphoteren grenzflächenaktiven Stoff, bei dem es sich zum Teil um Undecylenamidopropylbetain handelt, besteht, wobei die Zusammensetzung der folgenden Formel entspricht:

| | |
|---|---|
| Natriumlaurylethersulfat | 5 bis 15 % |
| Cocoamidopropylbetain | 2 bis 7 % |
| Undecylenamidopropylbetain | 1,5 bis 5,0 % |
| Herkömmliche Ingredienzien von Haarwaschmittelformulierungen und entmineralisiertes Wasser q.s.p. | 100 |
| (Gew.-% bezogen auf die Zusammensetzung). | |

**5.** Verwendung von Haarwaschmittelzusammensetzungen nach einem der vorhergehenden Ansprüche zur kosmetischen Behandlung der Bildung von Schuppen, die auf die Wirkung von *Pityrosporum ovalae* zurückzuführen sind.

## Claims

**1.** Aqueous antifungal and antidandruff shampoo compositions containing a washing base consisting of an anionic surfactant and an amphoteric surfactant, in which the anionic surfactant is taken, alone or as a mixture, from the group consisting of ($C_{10}$ to $C_{14}$)alkyl sulphates and ($C_{10}$ to $C_{14}$)alkyl ether sulphates and the amphoteric surfactant is taken, alone or as a mixture, from the group consisting of alkyl amphocarboxyglycinates, alkyl amphocarboxypropionates, alkyl amphodiacetates, alkyl amphodipropionates, alkyl amphoglycinates, alkyl amphopropionates, alkyl iminopropionates, alkyl iminodipropionates, alkyl amphopropyl sulphonates, alkylbetaines, alkylamidopropylbetaines, alkylsultaines and alkylamidopropylhydroxysultaines,
**characterized in that** some of the amphoteric surfactant is an undecylenamidopropylbetaine.

**2.** Aqueous shampoo compositions according to Claim 1, in which the anionic surfactant is a lauryl ether sulphate and the amphoteric surfactant is a cocoamidopropylbetaine.

**3.** Aqueous shampoo compositions according to Claim 1 or 2, in which the anionic and amphoteric components are distributed,
on the one hand, as regards the anionic components/amphoteric compounds equilibrium, according to the inequations:

$$12.5\% \leq An + \Sigma Amph \leq 17.6$$

$$5\% \leq An \leq 15$$

$$3.5\% \leq \Sigma Amph \leq 9.2$$

on the other hand, as regards the amphoteric compounds/undecylenic amphoteric component equilibrium according to the inequations:

$$3.5\% \leq \Sigma Amph \leq 9.2$$

$$2\% \leq Amph^* \leq 7$$

$$1.5\% \leq AmphU \leq 3.5$$

in which inequations
An is used for anionic components, Amph for amphoteric compounds,
ΣAmph represents all of the amphoteric compounds,
AmphU represents undecylenamidopropylbetaine,
Amph* represents the amphoteric components except for undecylenamidopropylbetaine.

**4.** Aqueous antifungal and antidandruff shampoo composition with a washing base consisting of an anionic surfactant and an amphoteric surfactant,
some of which is undecylenamidopropylbetaine, this composition corresponding to the formulation

| | |
|---|---|
| Sodium lauryl ether sulphate | 5 to 15% |
| Cocoamidopropylbetaine | 2 to 7% |
| Undecylenamidopropylbetaine | 1.5 to 5.0% |

(continued)

| | |
|---|---|
| Ordinary ingredients of shampoo formulations and demineralized water (per cent by weight relative to the composition). | qs 100 |

**5.** Use of the shampoo compositions according to any one of the preceding claims for the cosmetic treatment of the formation of dandruff due to the action of *Pityrosporum ovalae.*